# EUROPEAN PATENT APPLICATION

(11) **EP 2 058 031 A1**
(43) Date of publication of application: **13.05.2009**
(21) Application number: 08386024.7
(22) Date of filing: 11.11.2008
(51) Int. Cl.: A63B 22/00, A61N 5/06

(54) **Gymnastics machine with ultraviolet**

(30) Priority: 12.11.2007 GR 20070100681
(71) Applicant: Kamposos,, Dimitrios, 21 200 Argos (GR)
(72) Inventor: Kamposos,, Dimitrios, 21 200 Argos (GR)

(57) **Abstract**

Combination of electro heat (exposure of the athlete to infrared light) with simultaneous exercise in machines such as treadmill, bicycle, elliptic machines and generally any machine for muscle exercise with the aim of increasing muscular strength, losing weight, cures from muscular diseases, steatolysis.

Luminous ultraviolet radiance is produced by filamentous tungsten lamps which are in α metal reflex. In any conventional aerobics machine there are filamentous tungsten lamps which are in metal reflector in proper arrangement and on stable metal bases, on the right, on the left and behind the athlete, which produce great temperature, transmitting ultraviolet radiance which has nothing to do with ultraviolet or any other kind of radioactive radiance. As the athlete is performing the exercise, simultaneously surface heating is obtained by the infrared light of lamps with the increase of the number of white blood corpsules, faster metabolism and better circulation. The result of it is the faster and more effective burning of body fat, providing the ability to exclude (remove) big amounts of unwanted toxins from the human body.

## Description

### Infrared radiance to which the device refers

The device refers to combination of electro heat that is the exposure of the athlete to infrared light and simultaneous athletic exercise on conventional machine such as electronic or conventional treadmill sitting or stand-back bicycle, elliptical machines and any machine that trains muscles with the aim of augmenting muscular strength, losing weight, curing muscular diseases, steatolysis.

The heat is defined as a form of energy which is transmitted from one soma to another soma of different temperature.

Heat transfer is achieved in three ways:
1) Through convection
2) Through co-convection
3) Through radiation

Thermal radiation is a form of energy which is emitted from somas which are heated and it is transmitted through electromagnetic waves. Thermal energy is radiated in wave lengths from λ=0 to λ=∞. The radiation spread speed in void equals light speed. The radiated heat from a soma depends on the body temperature and on the nature of the surface of the radiated soma. In physical therapy, heat through radiation is achieved especially with infrared devices.

### Relevant level of the technique

Through regional temperature increase in parts of the body, the following are increased:
1) Blood f low and oxygenating
2) Enzymatic activity and metabolism
3) Faster neural conduct of stimuli
What is decreased in regions which are regionally heated through temperature increase are:
1) Muscular spasm and pain
2) Joints hardness
3) Inflammatory infiltration and edemas

### Technical problem to be solved

Nevertheless conventional techniques of physical exercise which already exist for fat loss have not given any visible results within short due time in body regions where unequal fat increase is presented in comparison with the rest of the body. Through conventional techniques for calories consumption and general control of body weight, high calories burning cannot be achieved in limited number of exercises. Moreover conventional techniques are not indicated as applicable to people who cannot perform exercise, yet wish an effective control of body weight and a shape maintenance programme, such as people who use wheel-chairs or who cannot serve themselves, or even those who do not have results while practicing an exercise programme.

### Device presentation

Ultra-violet light is an important source of energy which in combination with conventional aerobics promotes healing- an increase of the number of white blood-corpsules, faster metabolism, better circulation and detoxication.

We also know that the more a body is heated, the more ultra-violet waves it emits. Bright radiance may be emitted by various sources such as the sun, incandescent lamps or fluorescent lamps etc. The length waves of the bright radiance which are included in the visible spectrum are a small percentage of the electromagnetic spectrum which includes X-rays, ultraviolet radiance, infrared radiance and radio waves. The wave length of the visible spectrum for human eye varies from 0.4 to 0.7 micrometers (µm). Infrared radiance is emitted in wave lengths from 0.7 to 10² µm.

The power of generators which produce infrared radiation varies from 60-1.500 Watt (curative characteristics). Heat is emitted quickly and no warming up is required. Ultra-violet waves which are emitted in wave length close to 0.7µm, which is close to the visible spectrum by the luminous ultra violet radiation generators, can penetrate through skin to hypodermal tissue. These waves are usually absorbed by all layers of derma and epidermis. Consequently:
1) The waves of luminous ultraviolet radiation penetrate deeper than the waves of non luminous ultraviolet radiation.
2) Luminous ultraviolet radiation is more stimulating for sentient thews and for sweat production.
3) The heat caused by luminous ultraviolet radiation is perceived by someone less intensely than the heat of non luminous, because the waves of luminous ultraviolet radiation are absorbed by all derma layers.

Luminous ultraviolet radiation is produced by filamentous tungsten lamps which are situated in metal reflex. These lamps develop very high temperature.

The results of this surface heat are the following;
1) Regional vasodilatation and hyperemia.
2) Increased regional metabolism.
3) Partial vasodilatation in deep subject tissues.
4) Appeasement of sentient thews with mild heat.
5) Muscular loosening.
6) Regional ephidrosis.
7) Breathing and heart beating acceleration, presupposing it is applied for enough time. This happens because organism temperature is increased and organism, through raising breathing beat and heartbeat, tries to exclude exaggerated heat and preserve temperature in normal levels.

### Device advantages

The combination of ultraviolet radiation and physical exercise succeeds visible results in short time distance in regional body parts which present unequal fat increase in connection with the rest of the body. This technique was applied in different groups who took part in a program of calories consumption where burning of 600 to 2,400 calories was achieved within one operation in 30 minutes. Moreover energy consumption equal to a 6 to 9-mile route was observed, during one and only operation. It is also an important advantage of the method in which ultraviolet radiation and physical exercise are simultaneously combined that it can be widely applied to people who use wheelchair or cannot serve themselves, or even those who cannot achieve results through physical exercise application.

Athlete's metabolism accelerates through this technique, and the result of real physical exercise is achieved without it really existing. Ultraviolet rays can penetrate deep into fat tissue and react in molecular level, helping burning of body fat more effectively and providing the ability to exclude big amounts of unwanted toxins and wastes, it increases circulation, increasing the creation of new capillaries, which are additional blood vessels that replace the ones in damage and accelerate healing procedure providing additional oxygen and nutritional elements needed for healing.

Further achievements are the following:
1. collagen production stimulation
2. ATP release stimulation
3. increase of the activity of lymphatic system
4. increase of RNA and DNA composition
5. reduction of the stimulus for the neural tissue
6. stimulation of neurons activity

### Description of a way of application of the device

In any common aerobics machine which activates fat tissue metabolism in mild tension and long duration, such as an electronic or conventional treadmill, sitting or standing bicycle, elliptic machines or machines in general which drill muscles with the aim of increasing muscular strength, losing weight and cures from muscular diseases, there are filamentous tungsten lamps which are in metal reflector in proper arrangement and on stable metal bases, on the right, on the left and behind the athlete, which produce great temperature, transmitting ultraviolet radiance close to visible spectrum (λ∼0.7µm) which has nothing to do with ultraviolet or any other kind of radioactive radiance. As the athlete is performing conventional exercise, simultaneously with sweating surface heating is achieved by the ultraviolet light of the lamps with the simultaneous increase of the number of white blood corpsules, faster metabolism, better circulation and detoxication. The result is faster and more effective burning of fat tissue, presupposing that the following organic functions are increased:
1. regional vasodilatation and hyperemia
2. increased regional metabolism
3. partial vasodilatation in deep subject tissues
4. appeasement of sentient thews with mild heating
5. muscular release
6. regional sweating
7. breath and heartbeat acceleration

## Claims

1. Combination of device which has filamentous tungsten lamps which produce luminous ultraviolet radiation with gymnastics machines (one or more) of any kind of aerobics or not to increase muscular strength, weight loss, cures from muscular diseases or even steatolysis.

2. Combination according to 1., where filamentous tungsten lamps are around and within distance from the body of the athlete who practices with gymnastics machines.

3. Combination according to assumptions 1&2 where the gymnastics machine is electronic or not, treadmill.

4. Combination according to assumptions 1&2 where the gymnastics machine is a bicycle.

5. Combination according to assumptions 1&2 where the gymnastics machine is a sitting bicycle.

6. Combination according to assumptions 1&2 where the gymnastics machine is a STEP.

7. Combination according to assumptions 1&2 where the gymnastics machine is a vibration device through which the muscles drill by vibration.

8. Combination according to assumptions 1&2 where the gymnastics machine is an elliptic STEP.
